# EUROPÄISCHE PATENTANMELDUNG

(11) **EP 2 095 845 A2**
(43) Veröffentlichungstag der Anmeldung: **02.09.2009**
(21) Anmeldenummer: 08171892.6
(22) Anmeldetag: 17.12.2008
(51) Int. Cl.: A61Q 5/00, A61K 8/41, A61K 8/34, A61K 8/37

(54) **Neuartige tensidhaltige Haarpflegezubereitung zum Schutz blond gefärbten Haares vor dem Auswaschen der Färbung**

(30) Priorität: 21.01.2008 DE 102008005411
(71) Anmelder: Beiersdorf AG, 20253 Hamburg (DE)
(72) Erfinder: Bluck, Manuela, 21529, Kröppelshagen-Fahrendorf (DE); Oelrichs, Ilka, 25436, Tornesch (DE); Köhler, Manuela, 20144 Hamburg (DE)

(57) **Zusammenfassung**

Zubereitung zum Schutz gefärbter keratinischer Fasern vor dem Auswaschen der Farbe enthaltend Wasser, Pigment, kationisches Tensid, Bis-Diglycerylpolyacyladipat-2 (Bis-DGPAA) und eine strukturbildende Komponente.

## Beschreibung

Die Erfindung betrifft eine neuartige tensidhaltige Haarpflegezubereitung zum Schutz blond gefärbten Haares vor dem Auswaschen der Färbung.

Viele Menschen färben sich die Haare. Blond ist dabei eine oft bevorzugte Farbe. Blonde, aber auch andere Färbungen sind nicht dauerhaft, auch wenn dies oft angenommen wird. Es kommt zum Auswaschen der Färbung, wenn das gefärbte Haar gewaschen oder mit Haarpflegemitteln behandelt wird. Bei einem einzelnen Wasch- oder Behandlungsvorgang wird dies mit dem Auge meist nicht wahrgenommen, bei einer Vielzahl solcher Vorgänge dagegen schon.

Haarpflegemittel sind zumeist tensidhaltige Zubereitungen, die Stoffe enthalten, die auf das Haar aufziehen und seine Eigenschaften verändern. Ziel ist die Verbesserung der Kämmbarkeit, des Glanzes und mancher anderer für den Träger des Haares wichtiger Eigenschaften des Haares.

Der Stand der Technik kennt Haarpflegezubereitungen. EP1 197 200 offenbart wässrige Haarpflegemittel mit UV-Absorbern und hinsichtlich ihrer Größe bestimmten Glimmer/TiO₂-Partikeln. EP 0 887 067 offenbart kosmetische, auch haarkosmetische Zubereitungen mit Glimmerpartikeln, die hinsichtlich ihrer Größe bestimmt sind und mit TiO₂ beschichtet sind. WO99/13844 offenbart haarkosmetische Zubereitungen mit optischen Aufhellern.

Zwar werden im Stand der Technik einige Zubereitungen offenbart, die durch Lichtschutzmittel das Ausbleichen des Haares zu verhindern suchen. Über eine Verringerung der Farbauswaschneigung blond gefärbter Haare wird nichts offenbart.

Es hat sich nun für den Fachmann überraschend herausgestellt, dass Zubereitung zum Schutz gefärbter keratinischer Fasern vor dem Auswaschen der Farbe, auch vor dem Ausbleichen, enthaltend Wasser, Pigment, kationisches Tensid, Bis-Dilycerylpolyacyladipat-2 (Bis-DGPAA, das Handelsprodukt Softisan 649 eignet sich besonders) und eine strukturbildende Komponente den Nachteilen des Standes der Technik abhilft. Dadurch bleibt das schöne Blond gefärbter Haare länger blond. Die Farbe bleicht nicht aus, wird also nicht heller. Von Vorteil ist es, wenn als kationisches Tensid ein quaternäres Ammoniumsalz eingesetzt wird, besonders bevorzugt Cetyltrimethyammoniumchlorid (CTAC). CTAC wird vorteilhaft in einer Konzentration von 0,7 bis 2 Gew.-%, bevorzugt 1 Gew.-% eingesetzt. Statt CTAC kann auch ein Anteil Behenyltrimethylammoniumchlorid eingesetzt werden, insbesondere 0,07 bis 0,2 Gew.-%, bevorzugt 0,1 Gew.-% bezogen auf die Gesamtformulierung. Das quaternäre Ammoniumsalz pflegt das Haar: eine bessere Kämmbarkeit, glattere Haarstruktur, mehr bzw. schönerer Glanz, mehr Geschmeidigkeit, bessere Entwirrbarkeit werden erreicht. Von Vorteil ist es, wenn zusätzlich Glycerin enthalten ist. Dies verbessert die Sensorik und die Stabilität der Formel insbesondere in der Kälte. Von Vorteil ist es, wenn Glycerin in Konzentrationen von 5 bis 13 Gew.-% enthalten ist. Von Vorteil ist es, wenn die strukturbildende Komponente ein Verdicker und/oder ein Fettalkohol ist, besonders bevorzugt ein Fettalkohol, ganz besonders bevorzugt ein Fettalkohol mit einer Molmasse von 242 bis 285 g/mol ist. Besonders bewährt hat sich eine Mischung aus zwei Fettalkoholen: C16 und C18, insbesondere eine Mischung im Massen-Verhältnis 1:2 bis 2:1 ganz besonders bevorzugt 1:1. Diese strukturbildende Komponente ist besonders geeignet, die Viskosität einzustellen und führt zu der gewünschten Sensorik des Produktes bei der Applikation des Produktes. Von Vorteil ist es, wenn die strukturbildende Komponente in einer Konzentration von 2,9 bis 7 Gew.-%, besonders bevorzugt 4 bis 5 Gew.-% enthalten ist. Von Vorteil ist es, wenn die Konzentration an kationischem Tensid 0,1 bis 1 Gew.-% beträgt. Dies verbessert die Stabilität der Formel. Von Vorteil ist es, wenn die Zubereitung als Emulsion vorliegt. Zur Herstellung einer emulsionsförmigen erfindungsgemäßen Zubereitung ist es von besonderem Vorteil, wenn erfindungsgemäße Fettalkohole enthalten sind. Diese stabilisieren die Pigmentpartikel. Bei der Herstellung der Emulsionen gemäß der Erfindung ist es vorteilhaft, wenn zunächst Fett- und Wasserphase separat hergestellt, anschließend vereinigt und dispergiert werden uns anschließend die Pigmente zugegeben werden. Dabei werden die Pigmente als Feststoff über einen Zeitraum von etwa 3 - 8 Minuten gleichmäßig auf die Oberfläche des Bulks, also die Produktmasse gestreut. Dabei rührt die verwendete Küchenmaschine (Modell "Kitchen Aid") kontinuierlich und damit die Pigmente in das Produkt ein. Dies geschieht nach der Homogenisierung. Dies hat den Vorteil, dass die empfindlichen Pigmente nicht beschädigt werden und keine Agglomerate bilden. Von Vorteil ist es, wenn die Konzentration an Bis-DGPAA 1 bis 3 Gew.-%, bevorzugt 2 Gew.-% beträgt. Dies bewirkt eine reichhaltige Sensorik und trägt zur Stabilisierung emulsionsförmiger Zubereitungen bei. Von Vorteil ist es, wenn das Pigment eine mittlere Teilchengröße von 5 bis 100 µm aufweist. Dadurch sind die Partikel in der Formel gut sichtbar, ohne dass der Anwender sie als Partikel bei der Verteilung im Haar spürt. Von Vorteil ist es, wenn das Pigment/die Pigmente -es handelt sich ja im Endeffekt oft um eine Mischung verschiedener Pigmente - in einer Konzentrationen von 0, 7 bis 1 Gew.-% enthalten ist/sind. Von Vorteil ist es, wenn in einem Wellenlängenbereich von 400 - 700 nm, das Verhältnis des Durchschnitts der Reflexion von Pigment zu dem Durchschnitt der Reflexion der Haarfasern zwischen 5.0 und 6.5 liegt. Gut geeignet sind gelbe, goldene und kupferfarbene Pigmente. Von Vorteil ist es, wenn die Zubereitung zusätzlich ein Cellulosederivat gewählt aus der Gruppe Methylcellulosen, 2-Hydroxyethyl-, 2-Hydroxypropyl- oder 2-Hydroxybutyl-Gruppen sowie Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugt sind (Hydroxypropyl)methylcellulosen, (beispielsweise die unter der Handelsbezeichnung Methocel E4M bei der Dow Chemical Comp ) enthält. Von Vorteil ist es, wenn das Cellulosederivat Hydroxypropylmethylcellulose (HPMC) ist. HPMC gibt den Produkten einen glitschigen Griff, den Anwender gerne mögen. Von Vorteil ist es, wenn das Cellulosederivat in Konzentrationen von 0,1 bis 0,5 Gew.-% vorliegt. Von Vorteil ist es, wenn das Verhältnis zwischen der Einsatzkonzentration an kationischem Tensid zu strukturbildender Komponente 1: 2.6 bis 1:11 beträgt. Wenn zu wenig kationisches Tensid im Verhältnis zum Fettalkohol vorliegt, wird die Formel instabil und trennt sich in ihre Phasen. Von Vorteil ist es, wenn die Partikel sphärisch sind. Von Vorteil ist es, wenn die Partikel beschichtet sind. Von Vorteil ist es, wenn die Partikel mit mindestens einem Metalloxid, insbesondere Titandioxid, optional auch mit weiteren Metalloxiden beschichtet sind. Von Vorteil ist es, wenn die Zubereitung frei von anionischen Tensiden ist. Von Vorteil ist es, wenn Die Zubereitung bei der Anwendung des FMH-Tests eine Veränderung des L*-Wertes von höchstens 0,17 hervorruft. Die Erfindung umfasst auch die Verwendung einer der beschriebenen Zubereitungen zum Schutz des gefärbten Haares vor dem Auswaschen der Farbe. Insbesondere betrifft die Erfindung die Verwendung einer der beschriebenen Zubereitungen zum Schutz des blond gefärbten Haares vor dem Auswaschen der Farbe.

Die erfindungsgemäßen Zubereitungen können ohne kationische Polymere hergestellt werden.

Die erfindungsgemäßen Zubereitungen können ohne Emulgatoren (oberflächenaktive Verbindungen mit einem HLB-Wert von 3 bis 18, insbesondere 8 bis 18) hergestellt werden. Die erfindungsgemäßen Zubereitungen können ohne anionische Tenside (oberflächenaktive Verbindungen mit einem HLB-Wert von 13 bis 15) hergestellt werden.

Die erfindungsgemäßen Zubereitungen können ohne von Glycerin verschiedene Polyole hergestellt werden.

Die erfindungsgemäßen Zubereitungen können ohne Polyalkylenglycole (PEGs und/oder PPGs) hergestellt werden.

Bei der Erfindung werden anorganische mehrschichtige Partikel, die z. B. aus einer Schicht Mica, einer Schicht Titandioxid und einer oder mehrer Schichten eines Metalloxides bestehen eingesetzt. Beispiele sind Timiron Silk Gold, Flamenco Orange 320C, Colorona Passion Orange, Colorona Copper, Colorona Blackstar Red, Colorona Blackstar Gold, Colorona Blackstar Green, Colorona Passion Orange, Timiron Super Red, Timiron Gleamer Flake MP-45, Timiron Diamond Cluster MP-149, Timiron Superluster MP-101, Timiron Stardust MP-80, Timiron Super Copper, Timiron Blue MP-155, Timiron Starlight Blue, Timiron Super Blue, Timiron Silk Blue, Sericite S-100 (Black,S.), Mearl Mica SVA, Mica 217N-12, Mica Covasil 4.05, Mica S-12, Cloisonne NU-Antique Copper 340XB, Cloisonne Sparkle Copper 350 J, Timiron BF-1001, Colorona Bright Gold. Dabei wird die Farbe der Partikel wird passend zur Haarfarbe gewählt.

Diese Partikel werden in ein Haarpflegeprodukt (Spülung und Kur) eingearbeitet.

Der FMH-Tests misst u. a. eine Veränderung des L*-Wertes. Ermittelt werden die Farbwerte vor und nach der Bestrahlung bzw. vor und nach der Haarwäsche. Von den zu vermessenden Haarsträhnen werden die Farbwerte vor der Bestrahlung bzw. Wäsche ermittelt. Anschließend erfolgen die Prüfmusterapplikation, die Bestrahlung und die erneute Ermittlung der Farbwerte der Haarsträhnen. Die Messung der Farbwerte erfolgt mit dem portablen Spektralphotometer SP-62 der Fa. X-Rite. Als Untergrund dient eine homogen grau gefärbte PVC-Platte. Die Streuung der Farbwerte ist sehr gering, so dass unter Umständen das Signifikanzniveau auf 99 % angehoben werden kann. Je Prüfmuster werden mindestens 10 Haarsträhnen gemessen. Gleichzeitig werden 10 mit einem Placebo behandelte Haarsträhnen als Referenz gemessen. Verwendet werden gekürzte Standardhaarsträhnen. Je nach gewünschter Trennschärfe können auch mehr als 10 Haarsträhnen gemessen werden, es ist jedoch zu beachten, dass die Bestrahlungskammer nur Raum für maximal 22 Haarsträhnen bietet. Weniger als 10 Tressen je Prüfmuster sind nicht zulässig. Die Standardsträhne wurden mit L'oreal Recital Preference Nr. 8 gefärbt. Die gefärbten Strähnen wurden im Ultraschallbad jeweils 15 min im Ultraschallbad ausgewaschen. Die Studiendurchführung erfolgt im Indexverfahren, d. h. zunächst werden von den verwendeten Haarsträhnen die so genannten Nullwerte, das sind die Farbwerte der Haarsträhnen vor der Prüfmusterapplikation, als Standard ermittelt. Anschließend erfolgt die Applikation der Prüfmuster. Sodann werden die Haarsträhnen ggf. beidseitig bestrahlt. Nachfolgend werden erneut die Farbwerte aufgenommen. Diese bezeichnet man als Messwerte. Je nach Studienanforderungen wird die Prozedur wiederholt. Die Studiendurchführung ist detailliert in der Working Instruction 4 STU 252 Farbmessungen beschrieben.

Nach der Ermittlung der Null- und der Messwerte werden die absoluten Farbwerte einzeln ausgewertet.

Die Angabe des ΔE (absoluter Farbabstand) ist nur dann sinnvoll, wenn die Richtung der Farbänderung im Farbraum mit dargestellt wird, bzw. wenn die Farbänderung in nur eine Richtung verläuft.
Δ*L** = absolute Helligkeitsänderung
Δ*a** = absolute Änderung des rot-grün-Wertes
Δ*b** = absolute Änderung des blau-gelb-Wertes.

### Beispiele

Als CTAC wurde CTAC 6025 KC verwendet.
Als Bis-DGPAA wurde Softisan 649 verwendet
Als HPMC wurde Methocel E4M Premium verwendet
Als Cetearyl Alcohol + Behentrimonium Chloride wurde Incroquat Behenyl TMC-25 verwendet
Als Glycerin + Lactic Acid + PEG-40 Hydrogenated Castor Oil + Trideceth-9 + Theobroma Cacao Extract wurde Extrapone Cocao GW 604689 verwendet
Als Propylene Glycol + Lactic Acid + Glucose + Aesculus Hippocastanum Seed Extract wurde Extrapone Horse Chestnut 605510 verwendet.
Als Caprylic/Capric Triglyceride + Lilium Candidum wurde Oil Extract of Lys Flowers verwendet. Als Glycerin wurde Glycerol EP vegetable 99,7% verwendet.
Als Cetrimmonium Chlorid wurde CTAC 6025 KC verwendet.
Als Methylparaben wurde Nipagin M verwendet.
Als Propylparaben wurde Nipasol M verwendet.
Als Benzophenon-4 wurde Escalol 577 verwendet.
Als Oryzanol wurde Gamma Oryzanol verwendet.
Als Zitronensäure wurde Citric acid Monohydrat fine Granular 04 3277 6 verwendet.
Als Sodium Hydroxide wurde Natronlauge 45% techn. rein verwendet.
Als Helianthus Annuus wurde Sonnenblumenöl, raff. Ph. Eur verwendet.
Als Triticum Vulgare wurde Wheat Germ Oil RBD verwendet.
Als Prunus Armeniaca wurde Apricot Kernel Oil RBDW verwendet.
Als Arachis Hypogaea wurde Erdnussoel raff. DAB 9 verwendet.
Als Panthenol wurde Dex Panthenol 75% aq. Solution verwendet
Als Propylene Glycol + Aqua + Citric Acid + Trideceth-9 + Punica Granatum Fruit Juice + Bisabolol wurde Fruitapone Granatapfel B verwendet.
Als Macadamia Ternifolia wurde Macadamia Nut Oil CP RBD verwendet.
Als Pigmente wurden verwendet:
Timiron Silk Gold,
Flamenco Orange 320C,
Colorona Passion Orange,
Colorona Copper,
Colorona Blackstar Red,
Colorona Blackstar Gold,
Colorona Blackstar Green,
Timiron Super Red,
Timiron Gleamer Flake MP-45,
Timiron Diamond Cluster MP-149,
Timiron Superluster MP-101,
Timiron Stardust MP-80,
Timiron Super Copper,
Timiron Blue MP-155,
Timiron Starlight Blue,
Timiron Super Blue,
Timiron Silk Blue,
Mearl Mica SVA,
Mica 217N-12,
Cloisonne NU-Antique Copper 340 XB,
Cloisonne Sparkle Copper 350 J,
Timiron BF-1001,
Colorona Bright Gold.

| | Bsp.1 | Bsp.2 | Bsp.3 | Bsp.4 | Bsp.5 |
|---|---|---|---|---|---|
| **INCI** | **Menge [%]** | **Menge [%]** | **Menge [%]** | **Menge [%]** | **Menge [%]** |
| Aqua | add 100 | add 100 | add 100 | add 100 | add 100 |
| Glycerin | 12,000 | 10,000 | 7,000 | 9,000 | 13,000 |
| Cetearyl Alcohol | 4,700 | 4,300 | 3,500 | 2,900 | 6,500 |
| Cetrimonium Chloride | 3,800 | 3,500 | 4,500 | 4,300 | 4,100 |
| Bis-Diglyceryl Polyacyladipate-2 (Bis-DGPAA) | 2,000 | 2,000 | 2,000 | 2,000 | 2,000 |
| Methylparaben | 0,250 | 0,250 | 0,250 | 0,250 | 0,250 |
| Propylparaben | 0,100 | 0,100 | 0,100 | 0,100 | 0,100 |
| Benzophenone-4 | 0,250 | 0,250 | 0,250 | 0,250 | 0,250 |
| Hydroxypropyl Methylcellulose | 0,190 | 0,350 | 0,400 | 0,250 | 0,300 |
| Cetearyl Alcohol + Behentrimonium Chloride | 0,200 | 0,200 | 0,200 | 0,200 | 0,200 |
| Oryzanol | 0,050 | 0,050 | 0,050 | 0,050 | 0,050 |
| Citric Acid | q.s. | q.s. | q.s. | q.s. | q.s. |
| Sodium Hydroxide | q.s. | q.s. | q.s. | q.s. | q.s. |
| Parfum | q.s. | q.s. | q.s. | q.s. | q.s. |
| Helianthus Annuus | 0,010 | | | 0,010 | |
| Triticum Vulgare | 0,010 | | 0,010 | | |
| Glycerin + Lactic Acid + PEG-40 Hydrogenated Castor Oil + Trideceth-9 + Theobroma Cacao Extract | | | 0,010 | 0,010 | |
| Propylene Glycol + Lactic Acid + Glucose + Aesculus Hippocastanum Seed Extract | | 0,010 | | | |
| Prunus Armeniaca | | 0,250 | | | |
| Cl 77891 + Mica + Tin Oxide | | 0,400 | | | |
| Mica + Cl 77891 + Cl 77492 | | | | | |
| Mica + Cl 77891 | 0,70 | 0,500 | | | |
| Mica + Cl 77491 + Alumina | 0,25 | | | | |
| Mica + Cl 77491 | | | 0,350 | | |
| Cl 77499 + Mica | | | | 0,800 | |
| Mica + Cl 77499 | | | | | 0,650 |
| Mica + Cl 77491 | | | 0,450 | | |
| Mica + Cl 77891 | | | | | 0,150 |
| Mica + Cl 77891 | | | | 0,200 | |

| | Bsp.6 | Bsp.7 | Bsp.8 | Bsp.9 | Bsp.10 |
|---|---|---|---|---|---|
| **INCI** | **Menge [%]** | **Menge [%]** | **Menge [%]** | **Menge [%]** | **Menge [%]** |
| Aqua | add 100 | add 100 | add 100 | add 100 | add 100 |
| Glycerin | 10,000 | 10,000 | 7,000 | 12,000 | 8,000 |
| Cetearyl Alcohol | 3,900 | 6,000 | 3,900 | 3,100 | 4,500 |
| Cetrimonium Chloride | 4,000 | 3,900 | 4,200 | 4,100 | 3,600 |
| Bis-Diglyceryl Polyacyladipate-2 | 2,000 | 2,000 | 2,000 | 2,000 | 2,000 |
| Methylparaben | 0,250 | 0,250 | 0,250 | 0,250 | 0,250 |
| Propylparaben | 0,100 | 0,100 | 0,100 | 0,100 | 0,100 |
| Benzophenone-4 | 0,250 | 0,250 | 0,250 | 0,250 | 0,250 |
| Hydroxypropyl Methylcellulose | 0,250 | 0,420 | 0,390 | 0,280 | 0,220 |
| Cetearyl Alcohol + Behentrimonium Chloride | 0,200 | 0,200 | 0,200 | 0,200 | 0,200 |
| Oryzanol | 0,050 | 0,050 | 0,050 | 0,050 | 0,050 |
| Citric Acid | q.s. | q.s. | q.s. | q.s. | q.s. |
| Sodium Hydroxide | q.s. | q.s. | q.s. | q.s. | q.s. |
| Parfum | q.s. | q.s. | q.s. | q.s. | q.s. |
| Helianthus Annuus | 0,050 | | | | |
| Triticum Vulgare | 0,050 | | | 0,010 | |
| Glycerin + Lactic Acid + PEG-40 Hydrogenated Castor Oil + Trideceth-9 + Theobroma Cacao Extract | | | 0,010 | | |
| Propylene Glycol + Lactic Acid + Glucose + Aesculus Hippocastanum Seed Extract | | | 0,010 | | |
| Caprylic/Capric Triglyceride + Lil- | | | | | 0,010 |
| ium Candidum | | | | | |
| Arachis Hypogaea | | 0,150 | | 0,100 | |
| Panthenol | 0,130 | | | 0,130 | |
| Cl 77891 + Mica + Tin Oxide | 0,500 | | | | |
| Mica + Cl 77891 + Cl 77492 | 0,250 | 0,250 | | | |
| Mica + Cl 77491 | | | | 0,700 | |
| Mica + Cl 77891 | | 0,350 | | | |
| Mica + Cl 77891 + Tin Oxide | | | 0,600 | | |
| Cl 77891 + Mica | | | 0,400 | | |
| Mica + Cl 77891 + Tin Oxide | | | | | 0,400 |
| Cl 77891 + Mica + Tin Oxide | | | | | 0,400 |
| Mica | | | | 0,200 | |

| **INCI** | Bsp.11 | Bsp. 12 | Bsp. 13 | Bsp. 14 | Bsp.15 |
|---|---|---|---|---|---|
| Aqua | **Menge [%]** | **Menge [%]** | **Menge [%]** | **Menge [%]** | **Menge [%]** |
| Glycerin | add 100 | add 100 | add 100 | add 100 | add 100 |
| Cetearyl Alcohol | 8,690 | 11,000 | 7,000 | 9,000 | 10,000 |
| Cetrimonium Chloride | 5,700 | 5,000 | 4,000 | 3,000 | 5,700 |
| Bis-Diglyceryl Polyacyladipate-2 | 4,000 | 4,100 | 3,800 | 3,600 | 4,000 |
| Methylparaben | 2,000 | 2,000 | 2,000 | 2,000 | 2,000 |
| Propylparaben | 0,250 | 0,250 | 0,250 | 0,250 | 0,250 |
| Benzophenone-4 | 0,100 | 0,100 | 0,100 | 0,100 | 0,100 |
| Hydroxypropyl Methylcellulose | 0,250 | 0,250 | 0,250 | 0,250 | 0,250 |
| Cetearyl Alcohol + Behentrimonium Chloride | 0,250 | 0,370 | 0,290 | 0,360 | 0,250 |
| Oryzanol | 0,200 | 0,200 | 0,200 | 0,200 | 0,200 |
| Citric Acid | 0,050 | 0,050 | 0,050 | 0,050 | 0,050 |
| Sodium Hydroxide | q.s. | q.s. | q.s. | q.s. | q.s. |
| Parfum | q.s. | q.s. | q.s. | q.s. | q.s. |
| Helianthus Annuus | q.s. | q.s. | q.s. | q.s. | q.s. |
| Triticum Vulgare | 0,100 | | | | |
| Glycerin + Lactic Acid + PEG-40 Hydrogenated Castor Oil + Trideceth-9 + Theobroma Cacao Extract | 0,100 | | | 0,010 | |
| Propylene Glycol + Lactic Acid + Glucose + Aesculus Hippocastanum Seed Extract | | 0,010 | | 0,010 | 0,010 |
| Propylene Glycol + Aqua + Citric Acid + Trideceth-9 + Punica Granatum Fruit Juice + Bisabolol | | | 0,010 | | 0,010 |
| Caprylic/Capric Triglyceride + Lilium Candidum | | | 0,010 | | |
| Prunus Armeniaca | | 0,010 | | 0,010 | |
| Macadamia Ternifolia | | | | | 0,150 |
| Cl 77891 + Mica + Tin Oxide | | | | 0,130 | |
| Mica + Cl 77891 + Cl 77492 | 0,500 | | 0,400 | | |
| Mica + Cl 77891 | 0,250 | | | | |
| Mica + Cl 77491 | | | | | 0,100 |
| Mica + Dimethicone + Trimethylsiloxysilicate | | 0,900 | | | |
| Mica + Isopropyl Titanium Trüsostearate | | | 0,450 | | |
| Mica + Cl 77491 + Cl 77499 + Cl 77891 | | | | 0,600 | |
| Mica + Cl 77491 | | | 0,350 | | 0,350 |
| Mica + Cl 77891 + Silver Oxide | | 0,100 | | | |

| **INCI** | Bsp. 16 | Bsp.17 | Bsp. 18 | Bsp. 19 | Bsp. 20 |
|---|---|---|---|---|---|
| Aqua | **Menge [%]** | **Menge [%]** | **Menge [%]** | **Menge [%]** | **Menge [%]** |
| Glycerin | add 100 | add 100 | add 100 | add 100 | add 100 |
| Cetearyl Alcohol | 8,000 | 12,000 | 13,000 | 8,000 | 8,690 |
| Cetrimonium Chloride | 3,900 | 4,600 | 4,800 | 4,800 | 5,700 |
| Bis-Diglyceryl Polyacyladipate-2 | 3,800 | 3,900 | 4,400 | 4,500 | 4,000 |
| Methylparaben | 2,000 | 2,000 | 2,000 | 2,000 | 2,000 |
| Propylparaben | 0,250 | 0,250 | 0,250 | 0,250 | 0,250 |
| Benzophenone-4 | 0,100 | 0,100 | 0,100 | 0,100 | 0,100 |
| Hydroxypropyl Methylcellulose | 0,250 | 0,250 | 0,250 | 0,250 | 0,250 |
| Cetearyl Alcohol + Behentrimonium Chloride | 0,300 | 0,300 | 0,400 | 0,370 | 0,250 |
| Oryzanol | 0,200 | 0,200 | 0,200 | 0,200 | 0,200 |
| Citric Acid | 0,050 | 0,050 | 0,050 | 0,050 | 0,050 |
| Sodium Hydroxide | q.s. | q.s. | q.s. | q.s. | q.s. |
| Parfum | q.s. | q.s. | q.s. | q.s. | q.s. |
| Helianthus Annuus | q.s. | q.s. | q.s. | q.s. | q.s. |
| Triticum Vulgare | 0,010 | | 0,010 | | 0,010 |
| Glycerin + Lactic Acid + PEG-40 Hydrogenated Castor Oil + Trideceth-9 + Theobroma Cacao Extract | | | | | 0,010 |
| Propylene Glycol + Lactic Acid + Glucose + Aesculus Hippocastanum Seed Extract | | 0,010 | | | |
| Propylene Glycol + Aqua + Citric Acid + Trideceth-9 + | | 0,010 | | 0,010 | |
| Punica Granatum Fruit Juice + Bis-abolol | | | | | |
| Caprylic/Capric Triglyceride + Lilium Candidum | | | 0,010 | | |
| Prunus Armeniaca | 0,010 | | | 0,010 | |
| Arachnis Hypogaea | | | 0,200 | | |
| Panthenol | | 0,100 | | | |
| Mica + Cl 77891 + Cl 77492 | | | | | 0,500 |
| Mica + Cl 77891 | | | | 0,250 | 0,250 |
| Mica + Cl 77491 | | | 0,750 | | |
| Mica + Cl 77891 | | | | | |
| Mica + Cl 77891 | | 0,300 | | | |
| Cl 77891 + Mica | 0,200 | | | | |
| Mica + Cl 77891 | | | 0,150 | | |
| Mica + Laurel Lysine | 0,600 | | | 0,350 | |
| Mica + Isopropyl Titanium Triisostearate | | | | 0,350 | |
| Mica + Cl 77491 | | 0,500 | | | |

### Anwendung:

Das Produkt wird in das Haar einmassiert und anschließend nach einer optionalen Einwirkzeit von bis zu 10 min ausgewaschen.

## Patentansprüche

1. Zubereitung zum Schutz gefärbter keratinischer Fasern vor dem Auswaschen der Farbe enthaltend Wasser, Pigment, kationisches Tensid, Bis-Diglycerylpolyacyladipat-2 (Bis-DGPAA) und eine strukturbildende Komponente.

2. Zubereitung nach Patentanspruch 1 **dadurch gekennzeichnet, dass** als kationisches Tensid ein quaternäres Ammoniumsalz eingesetzt wird, besonders bevorzugt Cetyltrimethylammoniumchlorid (CTAC).

3. Zubereitung nach einem der vorangehenden Patentansprüche **dadurch gekennzeichnet, dass** zusätzlich Glycerin enthalten ist.

4. Zubereitung nach einem der vorangehenden Patentansprüche **dadurch gekennzeichnet, dass** Glycerin in Konzentrationen von 5 bis 13 Gew.-% enthalten ist.

5. Zubereitung nach einem der vorangehenden Patentansprüche **dadurch gekennzeichnet, dass** die Strukturbildende Komponente ein Verdicker und/oder ein Fettalkohol ist, besonders bevorzugt ein Fettalkohol, besonders bevorzugt ein Fettalkohol mit einer Molmasse von 242 bis 285 g/mol ist.

6. Zubereitung nach einem der vorangehenden Patentansprüche **dadurch gekennzeichnet, dass** die strukturbildende Komponente in einer Konzentration von 2,9 bis 7 Gew.-% enthalten ist.

7. Zubereitung nach einem der vorangehenden Patentansprüche **dadurch gekennzeichnet, dass** die Konzentration an kationischem Tensid 0,1 bis 1 Gew.-% beträgt.

8. Zubereitung nach einem der vorangehenden Patentansprüche **dadurch gekennzeichnet, dass** die Konzentration an Bis-DGPAA 1 bis 3 Gew.-% beträgt.

9. Zubereitung nach einem der vorangehenden Patentansprüche **dadurch gekennzeichnet, dass** sie als Emulsion vorliegt.

10. Zubereitung nach einem der vorangehenden Patentansprüche **dadurch gekennzeichnet, dass** das Pigment eine mittlere Teilchengröße von 5 bis 100 µm aufweist.

11. Zubereitung nach einem der vorangehenden Patentansprüche **dadurch gekennzeichnet, dass** das Pigment/die Pigmente in einer Konzentrationen von 0,7 bis 1 Gew.-% enthalten ist/sind.

12. Zubereitung nach einem der vorangehenden Patentansprüche **dadurch gekennzeichnet, dass** in einem Wellenlängenbereich von 400 bis 700 nm, das Verhältnis des Durchschnitts der Reflexion von Pigment zu dem Durchschnitt der Reflexion der Haarfasern zwischen 5.0 und 6.5 liegt.

13. Zubereitung nach einem der vorangehenden Patentansprüche **dadurch gekennzeichnet, dass** sie zusätzlich ein Cellulosederivat gewählt aus der Gruppe Methylcellulosen, 2-Hydroxyethyl-, 2-Hydroxypropyl- oder 2-Hydroxybutyl-Gruppen sowie Cellulosemischether, die neben einem dominierenden Gehalt an Methyl- zusätzlich 2-Hydroxyethyl-, 2-Hydroxypropyl- oder 2-Hydroxybutyl-Gruppen enthalten.

14. Zubereitung nach einem der vorangehenden Patentansprüche **dadurch gekennzeichnet, dass** das Cellulosederivat Hydroxpropylmethylcellulose ist.

15. Zubereitung nach einem der vorangehenden Patentansprüche **dadurch gekennzeichnet, dass** das Cellulosederivat in Konzentrationen von 0,1 bis 0,5 Gew.-% vorliegt.

16. Zubereitung nach einem der vorangehenden Patentansprüche **dadurch gekennzeichnet, dass** das Verhältnis zwischen der Einsatzkonzentration an kationischem Tensid zu strukturbildender Komponente 1: 2.6 bis 1:11 beträgt.

17. Zubereitung nach einem der vorangehenden Patentansprüche **dadurch gekennzeichnet, dass** die Partikel sphärisch sind.

18. Zubereitung nach einem der vorangehenden Patentansprüche **dadurch gekennzeichnet, dass** die Partikel beschichtet sind.

19. Zubereitung nach einem der vorangehenden Patentansprüche **dadurch gekennzeichnet, dass** die Partikel mit mindestens einem Metalloxid beschichtet sind.

20. Zubereitung nach einem der vorangehenden Patentansprüche **dadurch gekennzeichnet, dass** sie frei von anionischen Tensiden ist.

21. Zubereitung nach einem der vorangehenden Patentansprüche **dadurch gekennzeichnet, dass** sie bei der Anwendung des FMH-Tests eine Veränderung des L*-Wertes von höchstens 0,17 hervorruft.

22. Verwendung einer Zubereitung nach einem der vorangehenden Patentansprüche zum Schutz des gefärbten Haares vor dem Auswaschen der Farbe.
